# EUROPÄISCHE PATENTANMELDUNG

(11) **EP 4 468 308 A1**
(43) Veröffentlichungstag der Anmeldung: **27.11.2024**
(21) Anmeldenummer: 24177829.9
(22) Anmeldetag: 24.05.2024
(51) Int. Cl.: G16H 40/40, G16H 40/63, G16H 20/17, A61M 1/14

(54) **VERFAHREN ZUM BETREIBEN EINES DIALYSESYSTEMS**

(30) Priorität: 24.05.2023 DE 102023113669
(71) Anmelder: B. Braun Avitum AG, 34212 Melsungen (DE)
(72) Erfinder: KRIETEMEYER, Stephan, 34212 Melsungen (DE); ODERNHEIMER, Philipp, 34212 Melsungen (DE); LIEB, Nino, 34212 Melsungen (DE); GIESA, Jonas, 34212 Melsungen (DE)
(74) Vertreter: Maiwald GmbH

(57) **Zusammenfassung**

Die Erfindung betrifft ein Verfahren zum Betreiben eines Dialysesystems umfassend mehrere hydraulisch miteinander in Verbindung stehende Komponenten und eine Steuervorrichtung, wobei das Verfahren umfasst, Empfangen, durch die Steuervorrichtung, von Komponenten-Betriebsdaten von mindestens einer der Komponenten, wobei die Komponenten-Betriebsdaten zumindest Bedarfsdaten umfassen, die einen zu erwartenden Ressourcenverbrauch der Komponente für eine bevorstehende, durch die Komponente auszuführende Aufgabe angeben, und Bestimmen, durch die Steuervorrichtung, von Betriebsparametern zum koordinierten Betreiben der Komponenten derart, dass ein zu erwartender Ressourcenverbrauch des Dialysesystems optimiert wird. Das Bestimmen der Betriebsparameter umfasst, basierend auf den Komponenten-Betriebsdaten und basierend auf Ressourcenverfügbarkeitsdaten und/oder Aufgabenpriorisierungsdaten, für eine oder mehrere der Komponenten mindestens einen Betriebsparameter zu bestimmen.

## Beschreibung

Offenbart sind ein Verfahren zum Betreiben eines Dialysesystems, ein System umfassend ein Dialysesystem, ein Computerprogrammprodukt und ein computerlesbares Medium.

Bislang stehen verschiedene Komponenten von Dialysesystemen, hier auch als Medizinprodukte bezeichnet, nur in hydraulischer Verbindung miteinander und werden einzeln und in der Regel manuell programmiert.

Dadurch können Synergien ungenutzt bleiben, was zu einer Verschwendung von Ressourcen, nämlich Wasser, Energie und/oder Zeit führen kann.

Eine Aufgabe, die der Erfindung zugrunde liegt, besteht also darin, ein Verfahren zum Betreiben eines Dialysesystems bereitzustellen, das ermöglicht, Verschwendung von Ressourcen zu reduzieren.

Die Aufgabe wird durch den Gegenstand der unabhängigen Ansprüche gelöst. Bevorzugte Ausführungsformen finden sich in den abhängigen Ansprüchen.

Die Erfindung betrifft ein Verfahren zum Betreiben eines Dialysesystems umfassend mehrere hydraulisch miteinander in Verbindung stehende Komponenten und eine Steuervorrichtung. Das Verfahren umfasst ein Empfangen, durch die Steuervorrichtung, von Komponenten-Betriebsdaten von mindestens einer der Komponenten, wobei die Komponenten-Betriebsdaten zumindest Bedarfsdaten umfassen, die einen zu erwartenden Ressourcenverbrauch der Komponente für eine bevorstehende, durch die Komponente auszuführende Aufgabe angeben. Das Verfahren umfasst auch ein Bestimmen, durch die Steuervorrichtung, von Betriebsparametern zum koordinierten Betreiben der Komponenten derart, dass ein zu erwartender Ressourcenverbrauch des Dialysesystems optimiert wird. Das Bestimmen der Betriebsparameter umfasst, basierend auf den Komponenten-Betriebsdaten und basierend auf Ressourcenverfügbarkeitsdaten und/oder Aufgabenpriorisierungsdaten, für eine oder mehrere der Komponenten mindestens einen Betriebsparameter zu bestimmen.

Die Komponente(n) kann/können also mit anderen Worten der Steuervorrichtung die Komponenten-Betriebsdaten bereitstellen, die Bedarfsdaten umfassen. Das heißt, die Komponente(n) kann/können bei der Steuervorrichtung ihren Bedarf anmelden. Optional kann/können die Komponente(n) jeweils Betriebsmodus und Betriebsphase an die Steuervorrichtung melden. Die Komponenten-Betriebsdaten, insbesondere Bedarfsdaten, können als dynamische Daten, also beispielsweise situationsspezifische Daten, betrachtet werden, die von der/den Komponente(n) bereitgestellt werden. Das Verfahren der vorliegenden Offenbarung ermöglicht also besonders präzise Optimierungsergebnisse, da nicht ausschließlich auf irgendwo hinterlegte Daten, beispielsweise aus einer Datenbank, zurückgegriffen werden muss, die weder für die vorliegende Situation noch für die tatsächliche Komponente zu diesem Zeitpunkt spezifisch sind. Somit kann der Ressourcenverbrauch auch besonders effektiv reduziert werden.

Das Dialysesystem kann beispielsweise Teil eines Dialysezentrums oder einer Dialysepraxis sein.

Die Komponenten, auch als Medizinprodukte bezeichnet, können beispielsweise Dialysegeräte, Heißreinigungsvorrichtungen, Umkehrosmosegeräte, Konzentratmischanlagen oder dergleichen umfassen.

Dass die Komponenten hydraulisch miteinander in Verbindung stehen bedeutet, dass Flüssigkeit zwischen den Komponenten gefördert, beispielsweise gepumpt, werden kann. Die hydraulische Verbindung kann in Form von Schläuchen und/oder Rohren ausgebildet sein. Die hydraulische Verbindung kann direkt oder indirekt sein. Das Dialysesystem kann Ventile umfassen, die beispielsweise zum Einstellen eines Flusses durch das Dialysesystem verwendet werden können, beispielsweise automatisch ansteuerbare Ventile.

Die Steuervorrichtung kann integral mit einer der Komponenten ausgebildet sein oder separat von den Komponenten ausgebildet sein.

Die Steuervorrichtung kann zum Empfangen von Daten von den Komponenten mittels einer, insbesondere bi-direktionalen, Datenverbindung mit der jeweiligen Komponente ausgebildet sein. Die Datenverbindung kann drahtlos oder drahtgebunden sein.

Der Begriff Komponenten-Betriebsdaten ist breit zu verstehen und kann beispielsweise Daten umfassen, die den Betrieb einer Komponente betreffen, so zum Beispiel Daten, die die aktuellen und/oder geplanten Einstellungen von Betriebsparametern repräsentieren, insbesondere umfassend Daten, die einen aktuellen und/oder geplanten Betriebsmodus der Komponente und/oder des Dialysesystems repräsentieren, und/oder Daten, die den aktuellen Zustand der Komponente repräsentieren, beispielsweise Anzustand, Auzustand, oder Fehlerzustand. Die Komponenten-Betriebsdaten können auch ein zu erwartendes Ergebnis, beispielsweise einen erwarteten Output, umfassen.

Wie oben gesehen umfassen die Komponenten-Betriebsdaten zumindest Bedarfsdaten, die einen zu erwartenden Ressourcenverbrauch der Komponente für eine bevorstehende, durch die Komponente auszuführende Aufgabe angeben. Die Komponenten-Betriebsdaten können insbesondere die auszuführende Aufgabe angeben und den zugehörigen zu erwartenden Ressourcenverbrauch.

Die Bedarfsdaten können einen zu erwartenden Ressourcenverbrauch für eine oder mehrere Ressourcen angeben, beispielsweise einen Wasserverbrauch und einen Energieverbrauch. Die Bedarfsdaten müssen den zu erwartenden Ressourcenverbrauch nicht in einer bestimmten Darstellungsweise, beispielsweise bestimmten Einheiten, angeben. Es genügt, dass die Bedarfsdaten derart ausgebildet sind, dass anhand der Bedarfsdaten der Ressourcenverbrauch ableitbar ist, beispielsweise durch die Steuervorrichtung.

Die Steuervorrichtung verwendet die Komponenten-Betriebsdaten zum Bestimmen von Betriebsparametern zum koordinierten Betreiben der Komponenten. Für eine oder mehrere der Komponenten wird dabei mindestens ein Betriebsparameter bestimmt.

Der Begriff "Betriebsparameter" ist breit zu verstehen und kann alle Parameter umfassen, deren Wert den Betrieb einer Komponente betrifft. Beispielsweise kann das Ändern des Wertes eines oder mehrerer Betriebsparameter die Einstellungen der Komponente ändern. Die Betriebsparameter können beispielsweise einen oder mehrere Betriebsmodi der Komponente und/oder des Dialysesystems umfassen. Betriebsparameter können alternativ oder zusätzlich beispielsweise eine Auswahl und/oder eine Reihenfolge an durch die Komponente auszuführenden Ausgaben, einen Ausführungszeitpunkt für eine oder mehrere durch die Komponente auszuführende Aufgaben, und/oder Einstellungen der Komponente, insbesondere zu bestimmten Zeitpunkten, umfassen.

Koordiniertes Betreiben kann umfassen, dass durch verschiedene der durch die Komponenten durchzuführende Aufgaben zeitlich abgestimmt durchgeführt werden, insbesondere in einer bestimmten Reihenfolge und/oder zu bestimmten Zeitpunkten und/oder mit bestimmten Einstellungen der Komponente.

Die Betriebsparameter werden derart bestimmt, dass der zu erwartende Ressourcenverbrauch des (insbesondere gesamten) Dialysesystems optimiert wird. Für die Optimierung können bekannte Optimierungsverfahren verwendet werden. Der zu erwartende Ressourcenverbrauch des Dialysesystems kann beispielsweise der kumulative Ressourcenverbrauch für einen vorgegebenen Zeitraum und/oder für einen vorgegebenen Betriebsabschnitt, beispielsweise in Form einer Gruppe von Aufgaben, sein. Der zu erwartende Ressourcenverbrauch, der optimiert wird, kann den Verbrauch nur einer Ressource oder den Verbrauch mehrerer Ressourcen umfassen.

Die Optimierung kann unter Anwendung von Randbedingungen durchgeführt werden, beispielsweise Randbedingungen, die einen sicheren Betrieb und/oder einen verschleißarmen Betrieb und/oder einen effizienten Betrieb der Komponente gewährleisten und/oder die Anzahl von On/Off-Zyklen reduziert.

Wie oben gesehen wird/werden der/die Betriebsparameter basierend auf den Komponenten-Betriebsdaten und basierend auf Ressourcenverfügbarkeitsdaten und/oder Aufgabenpriorisierungsdaten bestimmt. Insbesondere wird/werden der/die Betriebsparameter basierend auf den Bedarfsdaten und basierend auf Ressourcenverfügbarkeitsdaten und/oder Aufgabenpriorisierungsdaten bestimmt. Anhand der Aufgabenpriorisierungsdaten kann für die verschiedenen anstehenden Aufgaben beispielsweise eine relative Priorität bestimmt werden. Anhand der Ressourcenverfügbarkeitsdaten kann für eine oder mehrere Ressourcen bestimmt werden, wie groß die Verfügbarkeit, insbesondere in Abhängigkeit von der Zeit, ist. Anhand der Bedarfsdaten und der Ressourcenverfügbarkeitsdaten kann geprüft werden, zu welchen Zeitpunkten bestimmte Aufgaben mit ihrem zugehörigen Bedarf idealerweise durchgeführt werden sollten, so dass die verfügbaren Ressourcen optimal verwendet werden können und ausreichend Ressourcen verfügbar sind.

So kann insbesondere das Verschwenden von verfügbaren Ressourcen oder das zusätzliche Bereitstellen von Ressourcen reduziert werden.

Außerdem können so auf einfache Weise Aufgaben effizient durchgeführt werden. Beispielsweise kann eine Aufgabe, die eine Komponente durchführt, zwar grundsätzlich in einem (größeren) Bereich von Temperaturen durchgeführt werden, aber in einem Teilbereich von Temperaturen besonders effizient durchgeführt werden. Zum Bereitstellen der Temperatur für diese Aufgabe kann die Wärme, die eine anderen Komponente beim Ausführen einer Aufgabe abgibt, als Ressource verwendet werden. Die Aufgaben der beiden Komponenten können derart koordiniert durchgeführt werden, dass die Aufgabe der einen Komponente dann durchgeführt wird, wenn die durch die andere Komponente abgegebene Wärme eine Durchführung in dem besagten Teilbereich ermöglicht. Andernfalls würde die Aufgabe der einen Komponente mit niedrigerer Effizienz oder aber mit eigens dafür zusätzlich aufgebrachter Heizleistung durchgeführt werden. Das koordinierte Betreiben kann also, wie oben gesehen, die Gesamteffizienz des Dialysesystems erhöhen und so kann der zu erwartende Ressourcenverbrauch optimiert werden.

Gemäß der vorliegenden Offenbarung kann das Bestimmen der Betriebsparameter ein Gruppieren von Anforderungen der Komponenten und/oder Priorisieren der auszuführenden Aufgaben umfassen.

Anforderungen können beispielsweise Bedarfsdaten umfassen oder von Bedarfsdaten abgeleitet sein, insbesondere den Ressourcenverbrauch umfassen oder von dem Ressourcenverbrauch abgeleitet sein. Anforderungen können gruppiert werden oder, mit anderen Worten, Gruppen zugeordnet werden. Beispielsweise können Anforderungen von verschiedenen Komponenten, die sich auf dieselbe Ressource beziehen, zusammen gruppiert werden. Für die jeweilige Gruppe kann optional eine kumulative, insbesondere systemweite, Anforderung ermittelt werden, beispielsweise ein kumulativer Bedarf einer bestimmten Ressource. Das Gruppieren der Anforderungen kann zu einer Bestimmung der Betriebsparameter herangezogen werden, insbesondere zum Festlegen einer Ressourcenverteilung auf die Komponenten.

Das Priorisieren kann umfassen, den bevorstehenden, durch die Komponenten auszuführenden Aufgaben anhand der Aufgabenpriorisierungsdaten zu priorisieren, beispielsweise ihnen jeweils eine Priorität zuzuordnen. Dazu kann das Verfahren umfassen, dass die Steuervorrichtung die bevorstehenden, durch die Komponenten auszuführenden Aufgaben bestimmt, beispielsweise anhand der Komponenten-Betriebsdaten. Ein Priorisieren der Aufgaben ermöglicht, verfügbare Ressourcen effizient einzusetzen, ohne ein Risiko zu erhöhen, dass kritische Prozesse unterversorgt sind.

Gemäß der vorliegenden Offenbarung kann das Bestimmen der Betriebsparameter ein Bestimmen eines zeitlichen Ablaufs der Durchführung der Aufgaben umfassen. Mit anderen Worten kann der Betrieb der Komponenten zeitlich koordiniert werden.

Insbesondere kann eine Reihenfolge der Durchführung der Aufgaben bestimmt werden und entsprechende Betriebsparameter gewählt werden. Beispielhaft kann für jede Aufgabe ein fester Zeitpunkt oder ein festes Intervall für die Durchführung bestimmt werden und als Betriebsparameter festgelegt werden. Alternativ oder zusätzlich können beim Bestimmen der Betriebsparameter die Betriebsparameter derart gewählt werden, dass das Abschließen einer Aufgabe das Starten einer anderen Aufgabe auslöst und/oder dass verschiedene Aufgaben mit einem bestimmten Zeitversatz gestartet werden.

So kann ein systemweit koordinierter Betrieb sichergestellt werden, der die Ressourcen besonders effektiv nutzt.

Gemäß der vorliegenden Offenbarung kann das Bestimmen der Betriebsparameter unter Berücksichtigung von Ressourcenverfügbarkeit erfolgen, insbesondere unter Berücksichtigung von Energieverfügbarkeit und/oder von Flüssigkeitsverfügbarkeit.

Die Ressourcenverfügbarkeit kann in den Ressourcenverfügbarkeitsdaten enthalten sein oder von Ressourcenverfügbarkeitsdaten abgeleitet werden. Insbesondere kann die Steuereinrichtung die Ressourcenverfügbarkeitsdaten von einer oder mehreren Komponenten des Dialysesystem, die Ressourcen zur Verfügung stellen, beispielsweise Speicher, Wasseraufbereiter oder Heizkomponenten, empfangen.

Die Betriebsparameter können derart bestimmt werden, dass optimiert wird, wie verfügbare Ressourcen eingesetzt werden. Insbesondere kann die Optimierung dahin gehen, dass die Ressourcen möglichst schnell oder möglichst vollständig verwenden werden. Die Verwendung hängt dabei wiederum von den anstehenden Aufgaben und entsprechendem erwarteten Ressourcenverbrauch und ggf. Priorisierung der Aufgaben ab.

Beispielsweise kann das Bestimmen umfassen, dass bei Verfügbarkeit von Flüssigkeit einer bestimmten Temperatur zunächst Aufgaben durchgeführt werden, die eine höhere Temperatur erfordern, und erst im Anschluss solche Aufgaben, bei denen die Temperatur niedriger sein kann. So wird beispielsweise durch die Reihenfolge der Aufgaben die Verwendung der Ressource optimiert.

Gemäß der vorliegenden Offenbarung kann das Bestimmen der Betriebsparameter unter Berücksichtigung einer Systemkonfiguration des Dialysesystems, insbesondere der relativen Anordnung der Komponenten und/oder der hydraulischen Verbindungen zwischen den Komponenten, erfolgen.

Beispielsweise kann das Bestimmen der Betriebsparameter unter Berücksichtigung einer Reihenfolge, in der die Komponenten hydraulisch verschaltet oder verschaltbar sind, erfolgen, insbesondere anhand vorhandener hydraulischer Verbindungselemente und/oder anhand von Schaltelementen zwischen den Komponenten.

Gemäß der vorliegenden Offenbarung kann das Bestimmen der Betriebsparameter eine zeitliche Synchronisierung der Komponenten-Betriebsdaten umfassen.

Die zeitliche Synchronisierung der Komponenten-Betriebsdaten kann umfassen, dass Betriebsdaten verschiedener Komponenten auf eine einheitliche Zeit synchronisiert werden und die so synchronisierten Betriebsdaten für das Bestimmen der Betriebsparameter verwendet werden. Beispielsweise können Betriebsparameter für einen koordinierten Betrieb bereitgestellt werden. Beispielsweise kann durch die Synchronisierung eine eindeutige relative zeitliche Einordnung der Betriebsdaten erfolgen. Das Verfahren ermöglicht, Komponenten mit ihrer jeweiligen Systemzeit und unabhängig von der Systemzeit der anderen Komponenten zu betreiben, und dennoch zeitliche Koordination zu ermöglichen.

Gemäß der vorliegenden Offenbarung kann das Bestimmen der Betriebsparameter umfassen, Ziel-Konflikte für das Betreiben des Dialysesystems basierend auf Komponenten-Betriebsdaten und/oder Ressourcenverfügbarkeitsdaten, und/oder Aufgabenpriorisierungsdaten, zu erkennen und eine automatische Konflikt-Behebung einzuleiten und/oder eine Benutzerausgabe zu veranlassen, die auf einen Ziel-Konflikt hinweist.

Ziel-Konflikte können beispielsweise beinhalten, dass vorhandene Ressourcen, beispielsweise ermittelt aus den Ressourcenverfügbarkeitsdaten, nicht ausreichen, um alle Aufgaben gemäß den Komponenten-Betriebsdaten und/oder Aufgabenpriorisierungsdaten auszuführen. In einem solchen Fall kann eine automatische, beispielsweise regelbasierte, Konflikt-Behebung eingeleitet werden. Beispielsweise kann ein bestimmter Ziel-Konflikt identifiziert werden, eine zugehörige Regel aus einer Vielzahl von Regeln, die für mögliche Ziel-Konflikte jeweils vorgeben, wie der Ziel-Konflikt aufgelöst werden soll, auswählen und den Ziel-Konflikt gemäß dieser Regel auflösen. Alternativ oder zusätzlich kann eine Benutzerausgabe, beispielsweise ein Hinweis auf einem Bildschirm oder ein akustisches oder optisches Warnsignal, veranlasst werden, die auf den Ziel-Konflikt hinweist. Der Benutzer kann daraufhin Schritte einleiten, um den Konflikt aufzulösen.

Gemäß der vorliegenden Offenbarung kann das Verfahren ein Empfangen, durch die Steuervorrichtung, von Daten von mindestens einem externen Informationssystem umfassen und das Bestimmen der Betriebsparameter unter Berücksichtigung der von dem externen Informationssystem empfangenen Daten erfolgen.

Das externe Informationssystem kann als Informationssystem betrachtet werden, das nicht Teil des Dialysesystems ist. Beispielsweise kann es sich um ein Krankenhausinformationssystem (KIS) oder um ein Informationssystem, das Komponentenspezifikationen bereitstellt, handeln. Insbesondere kann das externe Informationssystem räumlich getrennt vom Dialysesystem, insbesondere außerhalb eines Dialysezentrums, angeordnet sein. Das Empfangen der Daten kann über gängige Datenschnittstellen erfolgen.

Es wird ermöglicht, beispielsweise von einem KIS oder einem Komponentenhersteller-Informationssystem Informationen zu erhalten, die für den Betrieb mindestens einer der Komponenten relevant sind. Das Berücksichtigen solcher Daten kann die Auswahl der Betriebsparameter verbessern. Beispielsweise können solche Daten Aufschluss über mögliche Betriebsmodi oder Spielräume bei den Betriebsparametern geben, so dass ein Optimieren der Betriebsparameter zugunsten des Ressourcenverbrauchs möglich ist.

Gemäß der vorliegenden Offenbarung kann das Verfahren einen durch die Steuervorrichtung veranlassten Austausch von Daten zwischen mindestens zwei der Komponenten mittelbar über die Steuervorrichtung umfassen.

Die Steuervorrichtung kann beispielsweise veranlassen, dass Daten, die die Steuervorrichtung von einer der Komponenten empfangen hat, an eine andere der Komponenten übertragen werden. Der durch die Steuervorrichtung veranlasste Austausch von Daten zwischen zwei Komponenten kann unidirektional oder bidirektional sein. Der durch die Steuervorrichtung veranlasste Austausch von Daten kann in Form eines Multicastings der Daten, die die Steuervorrichtung von einer der Komponenten empfangen hat, an mehrere der anderen Komponenten erfolgen. Insbesondere kann es sich bei den Daten um Komponenten-Betriebsdaten handeln. So kann beispielsweise ein Anschalten, ein Umstellen, ein Ausschalten und/oder eine Fehlfunktion einer Komponente an die Steuervorrichtung gemeldet werden und, über die Steuervorrichtung, mittelbar an eine oder mehrere andere Komponenten gemeldet werden.

Gemäß der vorliegenden Offenbarung kann der Ressourcenverbrauch einen Energieverbrauch und/oder einen Wasserverbrauch und/oder einen Zeitbedarf umfassen.

Der Energieverbrauch kann beispielsweise die Energiemenge sein, die zum Abschließen der Aufgabe erforderlich ist. Der Energieverbrauch kann dabei beispielsweise den Verbrauch elektrischer Energie und/oder von Energie in Form von Wärme umfassen. Der Wasserverbrauch kann beispielsweise die Wassermenge sein, die zum Abschließen der Aufgabe erforderlich ist. Bei dem Wasserverbrauch kann es sich um den Verbrauch von einer bestimmten Art von Wasser, beispielsweise aufbereitetem Wasser, handeln. Der Zeitbedarf kann beispielsweise die Zeitdauer sein, die zum Abschließen der Aufgabe erforderlich ist. Weitere denkbare Ressourcen sind dem Wasser zuzusetzende Inhaltsstoffe, beispielsweise Elektrolyte, oder andere Flüssigkeiten als Wasser.

Gemäß der vorliegenden Offenbarung können zumindest manche der Komponenten nur hydraulisch miteinander in Verbindung stehen. Insbesondere können diese Komponenten keine direkte Datenverbindung aufweisen. Das heißt, diese Komponenten können nur mittelbar über die Steuervorrichtung Daten austauschen. Ein direkter Datenaustausch zwischen diesen Komponenten kann also nicht möglich sein. Dies kann eine verbesserte Sicherheit gewährleisten und außerdem ist es nicht erforderlich, kompatible Schnittstellen und Protokolle bereitzustellen.

Gemäß der vorliegenden Offenbarung können die Komponenten mindestens eine Umkehrosmose-Vorrichtung und/oder mindestens eine Konzentratmischanlage und/oder mindestens ein Heißreinigungssystem und/oder mindestens ein Dialysegerät umfassen.

Die Umkehrosmose-Vorrichtung kann zur (Dialyse-)Wasseraufbereitung ausgebildet sein. Die Umkehrosmose-Vorrichtung kann aufbereitetes Wasser als Ressource bereitstellen. Der Ressourcenverbrauch der Umkehrosmose-Vorrichtung kann, neben der Zeit, einen Wasserverbrauch, beispielsweise von verbrauchtem Wasser, und einen Energieverbrauch umfassen.

Die Konzentratmischanlage kann zum Anmischen von (Dialyse-)Konzentrat ausgebildet sein. Die Konzentratmischanlage kann Dialyse-Konzentrat als Ressource bereitstellen. Der Ressourcenverbrauch der Konzentratmischanlage kann, neben der Zeit, einen Wasserverbrauch, einen Energieverbrauch, einschließlich elektrischer Energie und Wärme, und Zusatz umfassen. Die Temperatur spielt dafür eine Rolle, wie schnell und wie gut das Konzentrat vermischt wird.

Das Heißreinigungssystem kann zum Heißreinigen von Behältern und Leitungen des Dialysesystems ausgebildet sowie zur Bereitstellung von heißem Wasser zur Desinfektion der Dialysegeräte sein. Ferner kann das Heißreinigungssystem erwärmtes Wasser zum Auflösen von Pulverkonzentraten in Konzentratmischanlagen bereitstellen. Der Ressourcenverbrauch des Heißreinigungssystems kann, neben der Zeit, einen Wasserverbrauch und einen Energieverbrauch, einschließlich elektrischer Energie und Wärme, umfassen.

Das Dialysegerät kann zum Durchführen der Dialyse an einem Patienten ausgebildet sein. Der Ressourcenverbrauch des Dialysegeräts kann, neben der Zeit, einen Verbrauch von Dialyse-Konzentrat, einen Wasserverbrauch und einen Energieverbrauch, einschließlich elektrischer Energie und Wärme, umfassen. Das Dialysegerät kann verbrauchtes Wasser als Ressource zur Verfügung stellen.

Wie sich daraus ergibt, konkurrieren die verschiedenen Komponenten um Ressourcen und stellen teilweise Ressourcen zur Verfügung, die von anderen Komponenten unter Umständen verwendet werden können, statt auf externe Ressourcen zuzugreifen. Somit ist ersichtlich, dass ein koordinierter Betrieb der Komponenten den Ressourcenverbrauch verbessern kann und dass es möglich ist, Betriebsparameter dahingehend zu optimieren, ausgehend von Informationen über anstehende Aufgaben und damit zusammenhängende Bedarfe, Prioritäten der Aufgaben und Ressourcen.

Das Verfahren der vorliegenden Erfindung kann insbesondere vollständig automatisch, insbesondere durch die Steuervorrichtung, durchgeführt werden, soweit nicht explizit anders angegeben.

Die vorliegende Erfindung stellt auch ein Dialysesystem mit mehreren hydraulisch verbundenen Komponenten und einer Steuervorrichtung umfasst, wobei die Steuervorrichtung mit den Komponenten jeweils mittels einer Datenverbindung verbunden ist, wobei das Dialysesystem zur Durchführung des Verfahrens gemäß der vorliegenden Offenbarung ausgebildet ist.

Insbesondere kann die Datenverbindung eine Datenverbindung sein, die einen bi-direktionalen Datenaustausch ermöglicht, also insbesondere Übertragen von Daten von der Steuervorrichtung an die Komponente und von der Komponente an die Steuervorrichtung.

Die Erfindung stellt auch ein das Dialysesystem umfassendes System bereit.

Das System kann auch ein vom Dialysesystem externes Informationssystem umfassen, beispielsweise ein Krankenhausinformationssystem (KIS) oder ein Informationssystem, das Komponentenspezifikationen bereitstellt. Insbesondere kann das externe Informationssystem räumlich getrennt vom Dialysesystem, insbesondere außerhalb eines Dialysezentrums, angeordnet sein.

Hinsichtlich der übrigen Merkmale und Vorteile wird auf die obigen Ausführungen verwiesen.

Die vorliegende Erfindung stellt auch ein Computerprogrammprodukt bereit, umfassend Befehle, die bewirken, dass das Dialysesystem der vorliegenden Offenbarung die Verfahrensschritte gemäß der vorliegenden Offenbarung, insbesondere wie oben beschrieben, ausführt.

Die vorliegende Erfindung stellt auch ein computerlesbares Medium bereit, auf dem das Computerprogrammprodukt der vorliegenden Offenbarung gespeichert ist.

Die Merkmale und Vorteile, die im Zusammenhang mit dem Verfahren beschrieben wurden, gelten entsprechend auch für das Dialysesystem, System, Computerprogrammprodukt und computerlesbare Medium.

Weitere Beispiele und Ausführungsformen werden im Folgenden anhand der Figuren erläutert. Dabei zeigen:
- Figur 1: eine schematische Darstellung eines Systems gemäß der vorliegenden Offenbarung;
- Figur 2: eine schematische Darstellung eines Verfahrens gemäß der vorliegenden Offenbarung;
- Figur 3: eine schematische Darstellung eines Systems gemäß der vorliegenden Offenbarung;
- Figur 4: eine schematische Darstellung eines Systems gemäß der vorliegenden Offenbarung.

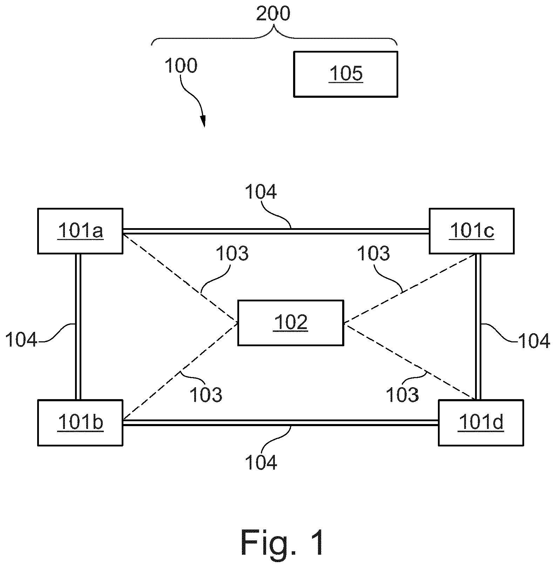

Figur 1 zeigt ein System 200 gemäß der vorliegenden Offenbarung umfassend ein Dialysesystem 100 gemäß der vorliegenden Offenbarung mit mehreren hydraulisch verbundenen Komponenten 101a, 101b, 101c, 101d und einer Steuervorrichtung 102, wobei die Steuervorrichtung mit den Komponenten jeweils mittels einer Datenverbindung 103, z.B. zum bi-direktionalen Datenaustausch, verbunden ist. Die hydraulische Verbindung ist mit dem Bezugszeichen 104 gekennzeichnet. Im vorliegenden Beispiel stehen die Komponenten nicht über eine Datenverbindung miteinander in Verbindung. Beispielsweise können sie ausschließlich hydraulisch miteinander verbunden sein.

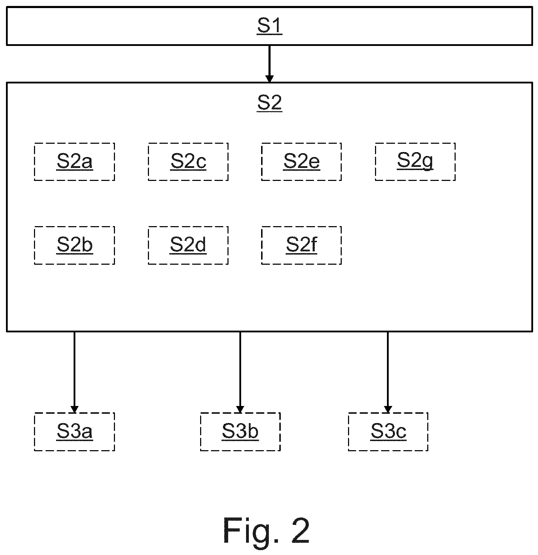

Das in Figur 1 gezeigte und oben beschriebene System kann zur Durchführung des Verfahrens gemäß der vorliegenden Offenbarung ausgebildet sein, insbesondere auch den unten im Zusammenhang mit Figuren 2 bis 4 beschriebenen Verfahrensschritten. Insbesondere kann die Steuervorrichtung ausgebildet sein, zumindest einen Teil der Verfahrensschritte, insbesondere alle Verfahrensschritte durchzuführen bzw. zu steuern.

Das System kann optional auch ein vom Dialysesystem externes Informationssystem 105 umfassen, beispielsweise ein Krankenhausinformationssystem (KIS) oder ein Informationssystem, das Komponentenspezifikationen bereitstellt. Insbesondere kann das externe Informationssystem räumlich getrennt vom Dialysesystem, insbesondere außerhalb eines Dialysezentrums, angeordnet sein.

In Figur 2 ist schematisch ein Verfahren zum Betreiben eines Dialysesystems, das mehrere hydraulisch miteinander in Verbindung stehende Komponenten, beispielsweise Medizinprodukte, und eine Steuervorrichtung umfasst, gemäß der vorliegenden Offenbarung dargestellt.

Das Verfahren umfasst, in Schritt S1, ein Empfangen von Komponenten-Betriebsdaten von mindestens einer der Komponenten durch die Steuervorrichtung. Die Komponenten-Betriebsdaten umfassend zumindest Bedarfsdaten, die einen zu erwartenden Ressourcenverbrauch der Komponente für eine bevorstehende, durch die Komponente auszuführende Aufgabe angeben. Der Ressourcenverbrauch kann beispielsweise einen Energieverbrauch und/oder einen Wasserverbrauch und/oder einen Zeitbedarf umfassen.

Das Verfahren umfasst, in Schritt S2, Bestimmen von Betriebsparametern zum koordinierten Betreiben der Komponenten derart, dass ein zu erwartender Ressourcenverbrauch des Dialysesystems optimiert wird, durch die Steuervorrichtung.

Das Bestimmen der Betriebsparameter umfasst, in Schritt S2a, basierend auf den Komponenten-Betriebsdaten und basierend auf Ressourcenverfügbarkeitsdaten und/oder Aufgabenpriorisierungsdaten, für eine oder mehrere der Komponenten mindestens einen Betriebsparameter zu bestimmen.

Optional kann das Bestimmen der Betriebsparameter, in Schritt S2b, ein Gruppieren von Anforderungen der Komponenten und/oder Priorisieren der auszuführenden Aufgaben umfassen. Optional kann das Bestimmen der Betriebsparameter, in Schritt S2c, ein Bestimmen eines zeitlichen Ablaufs der Durchführung der Aufgaben umfassen. Optional kann das Bestimmen der Betriebsparameter, in Schritt S2d, eine zeitliche Synchronisierung der Komponenten-Betriebsdaten umfassen.

Optional kann das Bestimmen der Betriebsparameter, in Schritt S2e, das Erkennen von Ziel-Konflikten für das Betreiben des Dialysesystems basierend auf Komponenten-Betriebsdaten und/oder Ressourcenverfügbarkeitsdaten und/oder Aufgabenpriorisierungsdaten umfassen. Das Bestimmen der Betriebsparameter kann weiter optional, in Schritt S2f, das Einleiten einer automatischen Konflikt-Behebung und/oder, in Schritt S2g, das Veranlassen einer Benutzerausgabe, die auf einen Ziel-Konflikt hinweist, umfassen.

Im vorliegenden Verfahren kann das Bestimmen der Betriebsparameter unter Berücksichtigung von Ressourcenverfügbarkeit erfolgen, insbesondere unter Berücksichtigung von Energieverfügbarkeit und/oder von Flüssigkeitsverfügbarkeit.

Alternativ oder zusätzlich kann im vorliegenden Verfahren das Bestimmen der Betriebsparameter unter Berücksichtigung einer Systemkonfiguration des Dialysesystems, insbesondere der relativen Anordnung der Komponenten und/oder der hydraulischen Verbindungen zwischen den Komponenten erfolgen.

Das Bestimmen der Betriebsparameter kann optional auch unter Berücksichtigung von von einem externen Informationssystem empfangenen Daten erfolgen. Das Verfahren kann optional in Schritt S3a das Empfangen dieser Daten umfassen.

Das Verfahren kann umfassen, dass die Steuervorrichtung im optionalen Schritt S3b von einer oder mehreren Komponenten des Dialysesystems Messdaten zum einströmenden Flüssigkeitsvolumen und zum ausströmenden Flüssigkeitsvolumen empfängt. Diese Daten können beim Bestimmen der Betriebsparameter verwendet werden. Insbesondere kann basierend auf diesen Daten eine Bilanzierung erfolgen.

Das Verfahren kann, im optionalen Schritt S3c, einen durch die Steuervorrichtung veranlassten Austausch von Daten zwischen mindestens zwei der Komponenten mittelbar über die Steuervorrichtung umfassen. Diese Daten können Bestimmen der Betriebsparameter verwendet werden.

Weitere Ausführungsbeispiele und Vorteile von Verfahren bzw. Systemen gemäß der vorliegenden Offenbarung werden nachfolgend beschrieben.

Bei derzeitigen Verfahren und Systemen werden typischerweise an jedem einzelnen Medizinprodukt eines Dialysesystems ein Zeitfenster für eine jeweils geplante Aktion einprogrammiert. Daraus ergeben sich verschiedene Probleme.

Ein Beispiel betrifft die Inline-Heißreinigung im Dialysesystem. Die Heizkapazität einer Inline-Heißreinigung ist durch die maximale Heizleistung der Heizer limitiert und kann nur eine begrenztet Anzahl von Dialysegeräten gleichzeitig mit heißem Wasser versorgen. Entnehmen zu viele Dialysegeräte gleichzeitig Heißwasser aus der Ringleitung, wird die für eine thermische Desinfektion notwendige Wassertemperatur nicht mehr erreicht, da die Heizkapazität der Heißreinigung überschritten wird.

Zudem betrifft dies auch Konzentratmischanlagen (CMS) des Dialysesystems. Diese benötigen Umkehrosmosewasser, um die in Satzform vorliegenden Konzentrate aufzulösen. Bei der Versorgung des Konzentratmischers wird unter Umständen kein von der Heißreinigung vorgewärmtes Umkehrosmsosewasser zur Verfügung gestellt, um die Anmischung des Dialysekonzentrats zu beschleunigen. Somit findet das Anmischen weniger effizient statt.

Während der Entnahme von Wasser kann bei derzeitigen Verfahren und Systemen zudem keine Leckage-Überwachung stattfinden, da die Entnahmemengen der Verbraucher nicht bekannt sind.

Allgemeint findet bei derzeitigen Verfahren und Systemen keine automatische Priorisierung der Aufgaben im Dialysesystem statt. In vielen Dialysezentren hat beispielsweise die sichere Desinfektion von Dialysegeräten vor der Anwendung beim Patienten höchste Priorität, vor der Desinfektion der Ringleitung, vor der Desinfektion der Umkehrosmose und letztlich auch vor der Anmischung von Dialysekonzentrat, da dieses auch in Kanistern zum Behandlungsort gebracht werden kann.

Es findet bei derzeitigen Verfahren und Systemen auch keine Bündelung von Aufgaben statt. Diese kann aber vorteilhaft sein, wie nachfolgend erläutert. Beispielsweise können Dialysegeräte zwar in (beispielsweise Fünfer-) Blöcken mit Heißwasser versorgt werden, das Ausspülen aller Geräte gleichzeitig kann aber beim Abkühlen der Ringleitung mit kaltem Wasser erfolgen (um Energie zu sparen). Als weiteres Beispiel kann der A0-Wert von 600 für die Ringleitung auch während der DI der Dialysemaschinen erreicht werden, ohne ein eigenes Heizfenster. Die thermische Desinfektion mit feuchter Hitze werden in Reinigungs- und Desinfektionsgeräten nach der Norm EN ISO 15883-1 (Reinigungs- und Desinfektionsgeräte - Teil 1: Allg. Anforderungen, Definitionen und Prüfungen) parametrisch über den A0-Wert definiert und kontrolliert. Der A0 Wert ist die Messung der aufgewendeten Energie (Temperatur/Zeit), die zeigt, ob der Desinfektionsprozess die gewünschte Letalität erreicht hat oder nicht. «A» ist dabei das Zeitäquivalent in Sekunden bei 80°C. Ein A0-Wert von 600 (z.B. 10 min / 80 °C oder 1 min / 90 °C) gilt als ausreichend zur Desinfektion von Bakterien und Pilzen, erfasst aber auch eine Reihe thermolabiler Viren sowie Noroviren.

Dies erlaubt eine Einsparung von Energie und Zeit. Während der Heißwasserentnahme der Dialysegeräte wird durch die kontinuierliche Beaufschlagung der Ringleitung mit heißem Wasser automatisch auch eine Desinfektion der Ringleitung erreicht.

Auch laufen bei derzeitigen Verfahren und Systemen Uhren der miteinander betriebenen Medizinprodukte (MP) oft nicht synchron, was beispielsweise das koordinierte Betreiben erschwert. Auch ein Abgleich der Fehlerlogs wird erschwert. Bei einer Umstellung zwischen Sommerzeit und Winterzeit kann es dadurch zudem ebenfalls zu Fehlern beim zeitsynchronen Abarbeiten von Programmen führen.

Zusammenfassend führt bei derzeitigen Verfahren und Systemen die manuelle Programmierung der nur hydraulisch miteinander in Verbindung stehenden Medizinprodukte zu Verschwendung von Ressourcen, nämlich Wasser, Energie und/oder Zeit, sowie anderen Problemen.

Das Verfahren und das System der vorliegenden Offenbarung erlauben es, zumindest manche der obigen Probleme zu beheben.

Gemäß einem Beispiel der vorliegenden Offenbarung sollen alle in einem Dialysezentrum nur hydraulisch miteinander in Verbindung stehenden Komponenten (Medizinprodukte), beispielsweise Umkehrosmosen, Konzentratmischanlagen, Heißreinigungen, Dialysegeräte, während der Betriebszeit durch eine zentrale Steuerung (beispielsweise durch die Steuervorrichtung) derart in ihren Betriebsmodi koordiniert werden, dass der Verbrauch an Ressourcen (Energie, Wasser) optimiert, Betriebszeiten minimiert und optional Leckagen sicher erkannt werden können.

Dazu können alle hydraulisch miteinander verbundenen Geräte bei der zentralen Steuerung ihre Bedarfe, beispielsweise für die dialysefreie Zeit, anmelden.

Die zentrale Steuerung kann die Anforderungen so gruppieren und priorisieren, dass alle Anforderungen gemäß ihrer Wichtigkeit ausgeführt werden, die Verbraucher so zugeschaltet werden, dass der Ressourcenverbrauch optimiert wird und unlösbare Zielkonflikte gemeldet werden, der Ablauf der gesteuerten Tätigkeiten protokolliert wird, der Energieverbrauch an der Energieverfügbarkeit ausgerichtet wird (Beispiel: gesteuerte Batchdesinfektion der Verbraucher unter Berücksichtigung der verfügbaren Heizleistung), und die Übermittelung von Betriebsdaten und Einstellungen an ein oder mehrere Netzwerkteilnehmer einen effizienten Betrieb ermöglicht.

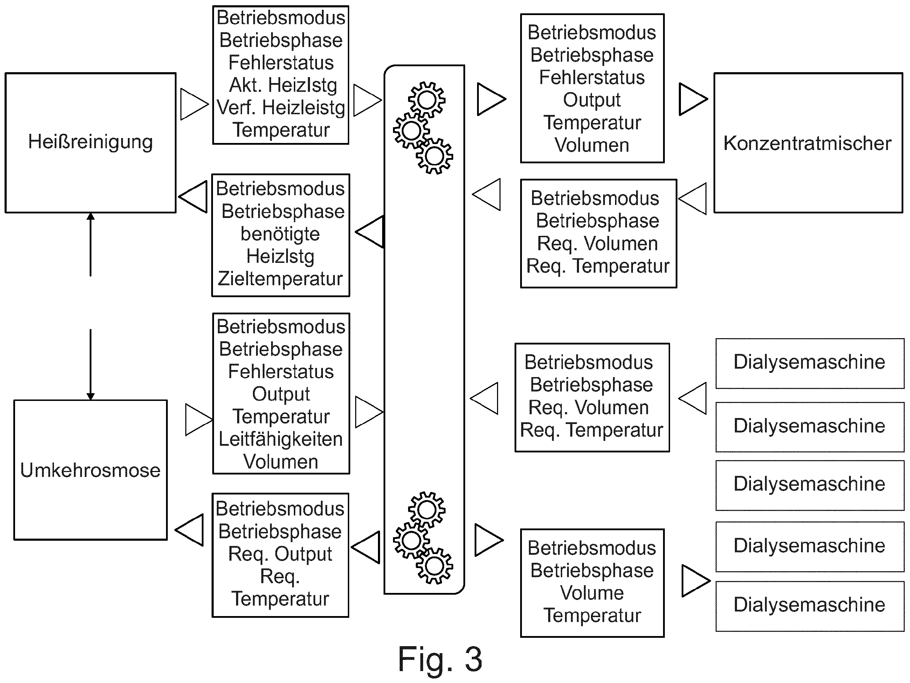

In Figur 3 ist eine schematische Darstellung der möglichen, in einem Dialysesystem gemäß der vorliegenden Offenbarung miteinander in Verbindung stehenden, aktiven Komponenten/Medizinprodukten gezeigt. Die zur zentralen Steuerung hinzeigenden Informationen werden von den angeschlossenen Komponenten/Medizinprodukten zur Verfügung gestellt. Die von der zentralen Steuerung herauszeigenden Information werden von der zentralen Steuerung Die von der zentralen Steuerung herauszeigenden Information werden von der zentralen Steuerung an Komponenten bereitgestellt.

Insbesondere sind hier die Komponenten "Heißreinigung", "Umkehrosmose", "Konzentratmischer" und "Dialysemaschine" beispielhaft gezeigt.

Die Komponenten können jeweils Betriebsmodus und Betriebsphase an die Steuerung melden. Zudem können auch Fehlerstatus, aktuelle Heizleistung (AKT HEIZLSTG, bspw. in kW), verfügbare Heizleistung (VERF HEIZLSTG, bspw. in kW) und Temperatur von der Heißreinigung gemeldet werden. Von der Umkehrosmose können zudem Fehlerstatus, Output (beispielsweise in Liter/Stunde), Temperatur, Leitfähigkeiten und Volumen gemeldet werden.

Vom Konzentratmischer und den Dialysemaschinen können zudem jeweils Volumenbedarf (REQ Volumen), beispielsweise an Wasser von der Umkehrosmose, und Temperaturbedarf (REQ Temperatur), beispielsweise Temperatur des Wassers aus der Umkehrosmose kommend, gemeldet werden.

Von der zentralen Steuerung können dann beispielsweise Betriebsmodus, Betriebsphase, Fehlerstatus, Output, Temperatur, Volumen an die Komponenten gemeldet werden. Außerdem können benötigte Heizleistung und Zieltemperatur an die Heißreinigung und/oder Volumenbedarf (REQ Volumen) und Temperaturbedarf (REQ Temperatur) an die Umkehrosmose gemeldet werden.

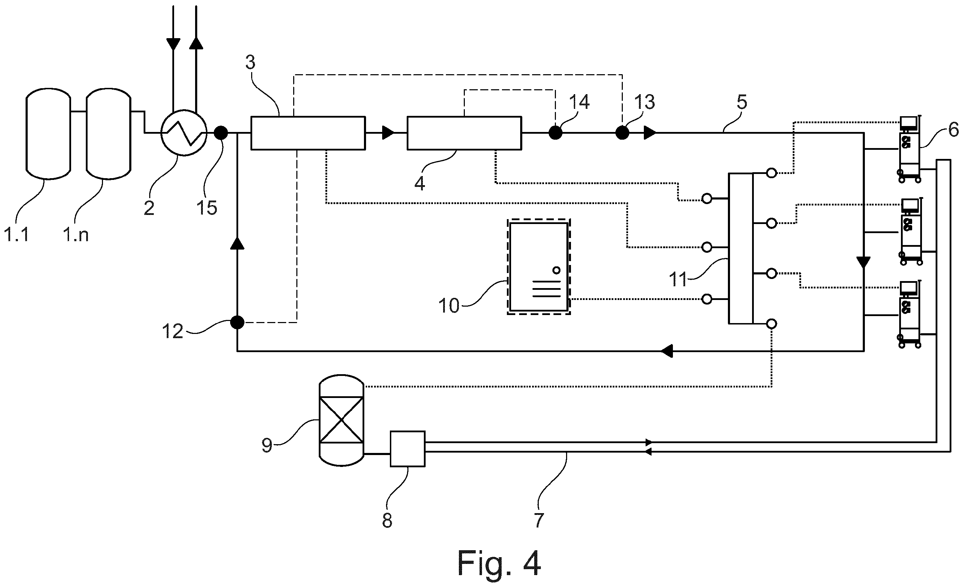

In Figur 4 ist eine schematische Darstellung der hydraulischen und informationstechnischen Verbindungen in einem beispielhaften Dialysezentrum umfassend ein beispielhaftes Dialysesystem gemäß der vorliegenden Offenbarung gezeigt. Das Dialysesystem umfasst in der Figur beispielhaft die folgenden Komponenten:
- 1.1: Vorbehandlungseinheit 1
- 1.n: Vorbehandlungseinheit n
- 2: Wärmetauscher
- 3: Umkehrosmose
- 4: Heißreinigung
- 5: Permeatringleitung
- 6: Verbraucher / Dialysegerät
- 7: Konzentratringleitung
- 8: Konzentratverteilanlage
- 9: Konzentratmischer
- 10: Steuerung
- 11: Netzwerk
- 12: Durchflußmesser Austritt Permeatringleitung
- 13: Durchflußmesser Eintritt Permeatringleitung
- 14: Temperatursensor Heißreinigung
- 15: Temperatursensor Austritt Wärmetauscher

Keines der oben gennannten Elemente, mit Ausnahme der Steuerung und ggf. geeigneter Datenverbindungen, ist zwingend erforderlich. Untergruppen der genannten Elemente können miteinander geeignet kombiniert werden. Insbesondere kann das Dialysesystem ohne Konzentratmischer 9 ausgebildet sein. Das Dialysesystem kann auch ohne Vorschaltung des Wärmetauschers sowie ohne den Temperatursensor Austritt Wärmetauscher 15 ausgebildet sein.

Im Folgenden wird ein beispielhaftes Verfahren gemäß der vorliegenden Offenbarung beschrieben. Bei der Beschreibung des Verfahrens wird rein beispielhaft auf das System, das in Figur 4 gezeigt ist, Bezug genommen. Das Verfahren kann aber auch mittels anderer geeigneter Systeme durchgeführt werden.

Gemäß dem Verfahren können alle über ein Netzwerk 11 mit einer Steuerung 10 verbundenen Komponenten (Medizinprodukte) ihre Bedarfe, beispielsweise Volumen, Temperatur, Betriebsmodus und/oder Betriebsphase, zentral bei der Steuerung anmelden. Nach Ermittlung der Ressourcenbedarfe, beispielsweise Permeatvolumen-Bedarfe, Temperaturen-Bedarfe, und/oder Zeitbedarfe, aller angeschlossenen Komponenten/Medizinprodukte kann die Steuerung beispielsweise eine optimale Reihenfolge der Zuschaltung aller Verbraucher ermitteln.

Beispielsweise kann die Ermittlung erfolgen, so dass zu keinem Zeitpunkt die maximale Heizkapazität der Heißreinigung 4 überschritten wird.

Alternativ oder zusätzlich kann die Ermittlung erfolgen, so dass Programmschritte in Heißdesinfektionsverfahren, welche kein heißes Wasser benötigen (insbesondere das Ausspülen der Dialysegeräte 6 nach chemothermischer Desinfektion), mit kaltem Wasser durchgeführt werden können.

Alternativ oder zusätzlich kann die Ermittlung erfolgen, so dass der Prozessschritt "Heißreinigung der Ringleitung" über eine A0 Wert Berechnung der erbrachten Desinfektionsleistung bei der Versorgung der Dialysegeräte mit heißem Wasser abgedeckt wird. Dabei findet Berücksichtigung, dass durch das Bereitstellen von heißem Wasser für die Desinfektion der Dialysegeräte gleichzeitig eine Erwärmung der Ringleitung stattfindet, so das während der Desinfektion der Dialysegeräte gleichzeitig eine Desinfektion der Ringleitung erzielt wird.

Alternativ oder zusätzlich kann die Ermittlung erfolgen, so dass die Konzentratmischanlage 9 mit Wasser einer vorbestimmten Temperatur (vgl. Sensor 14) gespeist wird, die geeignet ist, den Lösungsprozess des Salzes zu beschleunigen. Das fertig gemischte Konzentrat wird anschließend mit Hilfe einer Konzentratverteilanlage 8 und eine Konzentratringleitung 7 zum Dialysegerät 6 gefördert.

Das Netzwerk kann beispielhaft eine Solltemperatur für das Zulaufwasser 16 an den Wärmetauscher 2 senden, so dass die Umkehrosmose Membranen mit geringem Transmembrandruck betrieben und somit zusätzlich zur Verringerung der Nacherwärmung (siehe oben) die elektrische Leistungsaufnahme der Hochdruckpumpen der Umkehrosmosen verringert wird.

Optional kann aufgrund der angemeldeten Bedarfe der verbundenen Verbraucher durch eine Bilanzierung der Volumenströme am Eingang 13 und am Austritt 12 der Permeatringleitung 5 eine Leckageüberwachung ermöglicht werden.

Obwohl die Erfindung in den Figuren und der vorstehenden Beschreibung detailliert dargestellt und beschrieben ist, sind diese Figuren und Beschreibungen als beispielhaft und nicht einschränkend zu betrachten. Die Erfindung ist nicht auf die gezeigten Ausführungsformen beschränkt. In Anbetracht der vorstehenden Beschreibung und der Figuren wird es für den Fachmann offensichtlich sein, dass im Rahmen der Erfindung, wie sie in den Ansprüchen definiert ist, verschiedene Modifikationen vorgenommen werden können.

## Patentansprüche

1. Verfahren zum Betreiben eines Dialysesystems (100) umfassend mehrere hydraulisch miteinander in Verbindung stehende Komponenten (101a, 101b, 101c, 101d) und eine Steuervorrichtung (102), wobei das Verfahren umfasst,
Empfangen, durch die Steuervorrichtung, von Komponenten-Betriebsdaten von mindestens einer der Komponenten, wobei die Komponenten-Betriebsdaten zumindest Bedarfsdaten umfassen, die einen zu erwartenden Ressourcenverbrauch der Komponente für eine bevorstehende, durch die Komponente auszuführende Aufgabe angeben, und
Bestimmen, durch die Steuervorrichtung, von Betriebsparametern zum koordinierten Betreiben der Komponenten derart, dass ein zu erwartender Ressourcenverbrauch des Dialysesystems optimiert wird,
wobei das Bestimmen der Betriebsparameter umfasst, basierend auf den Komponenten-Betriebsdaten und basierend auf Ressourcenverfügbarkeitsdaten und/oder Aufgabenpriorisierungsdaten, für eine oder mehrere der Komponenten mindestens einen Betriebsparameter zu bestimmen.

2. Verfahren nach Anspruch 1, wobei das Bestimmen der Betriebsparameter ein Gruppieren von Anforderungen der Komponenten und/oder Priorisieren der auszuführenden Aufgaben umfasst.

3. Verfahren nach einem der vorangegangenen Ansprüche, wobei das Bestimmen der Betriebsparameter ein Bestimmen eines zeitlichen Ablaufs der Durchführung der Aufgaben umfasst.

4. Verfahren nach einem der vorangegangenen Ansprüche, wobei das Bestimmen der Betriebsparameter unter Berücksichtigung von Ressourcenverfügbarkeit erfolgt, insbesondere unter Berücksichtigung von Energieverfügbarkeit und/oder von Flüssigkeitsverfügbarkeit.

5. Verfahren nach einem der vorangegangenen Ansprüche, wobei das Bestimmen der Betriebsparameter unter Berücksichtigung einer Systemkonfiguration des Dialysesystems, insbesondere der relativen Anordnung der Komponenten und/oder der hydraulischen Verbindungen zwischen den Komponenten erfolgt.

6. Verfahren nach einem der vorangegangenen Ansprüche, wobei das Bestimmen der Betriebsparameter eine zeitliche Synchronisierung der Komponenten-Betriebsdaten umfasst.

7. Verfahren nach einem der vorangegangenen Ansprüche, wobei das Bestimmen der Betriebsparameter umfasst, Ziel-Konflikte für das Betreiben des Dialysesystems basierend auf Komponenten-Betriebsdaten und/oder Ressourcenverfügbarkeitsdaten, und/oder Aufgabenpriorisierungsdaten, zu erkennen und eine automatische Konflikt-Behebung einzuleiten und/oder eine Benutzerausgabe zu veranlassen, die auf einen Ziel-Konflikt hinweist.

8. Verfahren nach einem der vorangegangenen Ansprüche, wobei das Verfahren ein Empfangen, durch die Steuervorrichtung, von Daten von mindestens einem externen Informationssystem umfasst und das Bestimmen der Betriebsparameter unter Berücksichtigung der von dem externen Informationssystem empfangenen Daten erfolgt.

9. Verfahren nach einem der vorangegangenen Ansprüche, umfassend einen durch die Steuervorrichtung veranlassten Austausch von Daten zwischen mindestens zwei der Komponenten mittelbar über die Steuervorrichtung.

10. Verfahren nach einem der vorangegangenen Ansprüche, wobei der Ressourcenverbrauch einen Energieverbrauch und/oder einen Wasserverbrauch und/oder einen Zeitbedarf umfasst.

11. Verfahren nach einem der vorangegangenen Ansprüche, wobei zumindest manche der Komponenten nur hydraulisch miteinander in Verbindung stehen.

12. Verfahren nach einem der vorangegangenen Ansprüche, wobei die Komponenten mindestens eine Umkehrosmose-Vorrichtung und/oder mindestens eine Konzentratmischanlage und/oder mindestens ein Heißreinigungssystem und/oder mindestens ein Dialysegerät umfassen.

13. Dialysesystem (100) mit mehreren hydraulisch verbundenen Komponenten (101a, 101b, 101c, 101d) und einer Steuervorrichtung (102), wobei die Steuervorrichtung mit den Komponenten jeweils mittels einer Datenverbindung verbunden ist, wobei das Dialysesystem zur Durchführung des Verfahrens nach einem der vorangegangen Ansprüche ausgebildet ist.

14. Computerprogrammprodukt, umfassend Befehle, die bewirken, dass das Dialysesystem des Anspruchs 13 die Verfahrensschritte nach einem der Ansprüche 1 bis 12 ausführt.

15. Computerlesbares Medium, auf dem das Computerprogramm nach Anspruch 14 gespeichert ist.
